(19) Europäisches Patentamt
European Patent Office
Office européen des brevets

(11) Publication number: 0 234 946
A2

# (12) EUROPEAN PATENT APPLICATION

(21) Application number: 87301740.4

(22) Date of filing: 27.02.87

(51) Int.Cl.³: C 07 K 5/06
A 61 K 37/64

(30) Priority: 28.02.86 GB 8605049
28.08.86 GB 8620767

(43) Date of publication of application:
02.09.87 Bulletin 87/36

(84) Designated Contracting States:
AT BE CH DE ES FR GB GR IT LI LU NL SE

(71) Applicant: THE WELLCOME FOUNDATION LIMITED
183-193 Euston Road
London NW1 2BP(GB)

(72) Inventor: Allan, Geoffrey
The Wellcome Foundation Ltd. Langley Court
Beckenham Kent(GB)

(72) Inventor: Hardy, George William
The Wellcome Foundation Ltd. Langley Court
Beckenham Kent(GB)

(72) Inventor: Bull, Donald
The Wellcome Foundation Ltd. Langley Court
Beckenham Kent(GB)

(72) Inventor: Mills, Gail
The Wellcome Foundation Ltd. Langley Court
Beckenham Kent(GB)

(72) Inventor: Lee, Grahame Roy
The Wellcome Foundation Ltd. Temple Hill
Dartford Kent(GB)

(74) Representative: Garrett, Michael et al,
The Wellcome Foundation Limited Group Patents and Agreements Langley Court
Beckenham Kent BR3 3BS(GB)

## (54) New compounds.

(57) The present invention is concerned with compounds of formula (I), physiologically acceptable salts thereof, formulations containing them, their preparation and their use in medicine.

In formula (I)

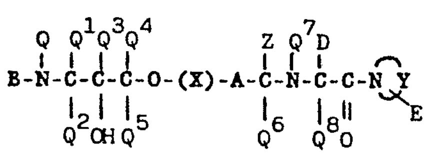

(I)

wherein:

Q and $Q^1$ - $Q^8$ are hydrogen;

A is a group of formula $-CONH(CH_2)_n-$, where n is 4 and $Q^9$ is hydrogen;

B is an isopropyl group;

E is a carboxyl group;

Z is a carboxyl or $C_{2-5}$ carboalkoxy group;

D is a methyl group;

(X) is a naphthyl or indolyl ring system; and

–N Y is a pyrrolidinyl ring;

and physiologically acceptable salts thereof;

with the condition that when (X) is a naphthyl ring system, the compound of formula (I) is:

N-(1(S)-carboxy-5- [5-(2(R,S)-hydroxy -3-isopropylaminopropoxy) naphth-2- ylcarboxamido] pentyl)-(R,S)-alanyl-(S)- proline, or

N-(1(S)- carboxy-5- [8-(2(R,S)- hydroxy-3-isopropylaminopropoxy) naphth -2-ylcarboxamido] pentyl)-(R,S)-alanyl-(S)- proline,

or a half ester (wherein Z is $C_{2-5}$ carboalkoxy) of either thereof, and when (X) is an indolyl ring system the compound of formula (I) is

N-(1(S)-carboxy-5- [4-(2(S)- hydroxy-3-isopropylaminopropoxy) -1*H*-indol -2-yl-carboxamido] pentyl)-(S)-alanyl-(S)- proline, or a half ester (wherein Z is $C_{2-5}$ carboalkoxy) thereof.

EP 0 234 946 A2

## NEW COMPOUNDS

This invention relates to new chemical compounds, their preparation, compositions containing them and their use in medicine.

Compounds which exhibit β-andrenergic blocking activity, for example as described in U.S. Patent 3,705,907, are widely used in the therapy of hypertensive states. Another class of agents used for the treatment of hypertension are inhibitors of angiotensin converting enzyme (ACE), for example, as described in European Patent 0012401 A1. We now believe that compounds which are simultaneously both β-blockers and ACE inhibitors are especially useful for the treatment and prophylaxis of hypertension and other conditions.

Our European Patent Application No. 85306144.8 discloses novel compounds of formula (I) (as defined hereinbelow) and their physiologically acceptable salts as exhibiting both β-blockade and ACE inhibitory activity.

In formula (I)

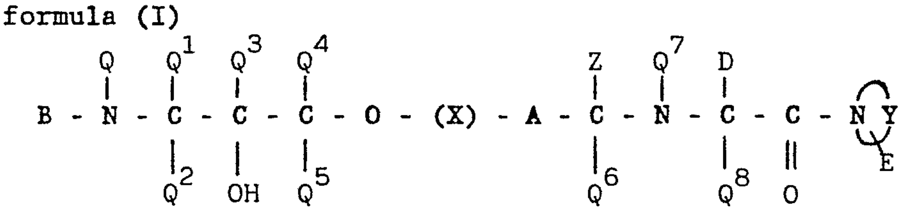

(I)

Q and $Q^1$-$Q^8$ are atoms or groups independently selected from hydrogen and $C_{1-4}$ alkyl;

A is a group of formula $-(CO)_k-(NQ^9)_m-(CH_2)_n-$ where k and m are either both 0 or both 1, n is from 1 to 6, $Q^9$ is selected from hydrogen and $C_{1-4}$ alkyl, and any of the $-(CH_2)_n-$ groups are independently optionally substituted by one or two $C_{1-4}$ alkyl groups;

B is a $C_{1-6}$ alkyl group;

E and Z are groups independently selected from carboxyl and carboxyl derivatives, e.g. esters;

D is hydrogen or a $C_{1-6}$ alkyl group which is optionally substituted by an amino group;

(X) is a benzene ring or a naphthyl or indolyl ring system any of which is optionally substituted in any position by one or more substituents independently selected from $C_{1-4}$ alkyl (itself optionally substituted by one or more halogen atoms), $C_{1-4}$ alkoxy, halo, nitro, amino, carboxy, $C_{1-4}$ alkoxycarbonyl and hydroxy; and

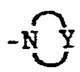 is a pyrrolidinyl, oxazolidinyl or thiazolidinyl ring, or a ring system selected from indolinyl, quinolinyl and tetrahydroquinolinyl,

and the formula (I) also includes the physiologically acceptable salts thereof.

We have now discovered that certain individual compounds of formula (I), and their physiologically acceptable salts, are particularly advantageous -blockers and ACE inhibitors.

The present invention thus provides the following three individual compounds, namely:

N-(1(S)-Carboxy-5-[5-(2(R,S)-hydroxy-3-isopropylaminopropoxy)naphth-2-y-lcarboxamido]pentyl)-(R,S)-alanyl-(S)-proline,

N-(1(S)-Carboxy-5-[8-(2(R,S)-hydroxy-3-isopropylaminopropoxy)naphth-2-y-lcarboxamido]pentyl)-(R,S)-alanyl-(S)-proline, and

N-(1(S)-Carboxy-5-[4-(2(S)-hydroxy-3-isopropylaminopropoxy)-1H-indol-2-ylcarboxamido]pentyl)-(S)-alanyl-(S)-proline,

and their half esters (wherein Z is $C_{2-5}$ carboalkoxy),

which are hereinafter termed the compounds of the invention, and their physiologically acceptable salts.

Of these, N-(1(S)-carboxy-5-[4-(2(S)-hydroxy-3-isopropylaminopropoxy)-1H-indol-2-ylcarboxamido]pentyl-(S)-alanyl-(S)-proline, its half esters (as defined above), and their pharmaceutically acceptable salts are particularly preferred by virtue of their especially advantageous combination of ACE-inhibitory and β-blocking properties.

The term "N-(1(S)-carboxy-5-[4-(2(S)-hydroxy-3-isopropylaminopropoxy)-1H-indol-2-ylcarboxamido]-pentyl-(S)-alanyl-(S)-proline" is used herein to describe the (S), (S), (S), (S)-isomer of N-(1-carboxy-5-[4-(2-hydroxy-3-isopropylaminopropoxy)-1H-indol-2-ylcarboxamido]pentyl-alanyl-proline when substantially free of other optical isomers or any mixture of said isomer with other optical isomers which advantageously contains at least 50% W/W, preferably at least 90% W/W, most preferably at least 95% W/W, of the former isomer.

The present invention also extends to the non-physiologically acceptable salts of the compounds of the invention, such as may be used as precursors in the preparation of the compounds of formula (I) and their physiologically acceptable salts.

The physiologically acceptable salts of compounds of the invention include salts derived from organic and inorganic acids as well as from bases. Salts derived from acids include the acetate, adipate, alginate, aspartate, benzoate, benzenesulphonate, bisulphate, butyrate, citrate, camphorate, camphorsulphonate, cyclopentanepropionate, digluconate, dodecylsulphate, ethanesulphonate, fumarate, glucoheptanoate, glycerophosphate, hemisulphate, heptanoate, hexanoate, hydrochloride, hydrobromide, hydroiodide, 2-hydroxyethanesulphonate, lactate, maleate, methanesulphonate, 2-naphthalenesulphonate, nicotinate, oxalate, palmoate, pectinate, persulphate, 3-phenylpropionate, pivalate, propionate, succinate, tartrate, thiocyanate, tosylate, and undecanoate.

Base salts include ammonium salts, alkali metal salts such as sodium and potassium salts, alkaline earth metal salts such as calcium and magnesium salts, salts with organic bases such as dicyclohexylamine salts, N-methyl-D-glucamine, and salts with amino acids such as arginine and lysine.

Quarternary ammonium salts can be formed where a nitrogen-containing group is present, for example by reaction with lower alkyl halides, such as methyl, ethyl, propyl, and butyl chlorides, bromides and iodides; dialkyl sulphates, long chain halides such as decyl, layryl, myristyl and stearyl chlorides, bromides and iodides, aralkyl halides like benzyl and phenethyl bromides.

In another aspect, the present invention provides compounds of the invention and physiologically acceptable salts thereof for use in medicine (including for the manufacture of therapeutic agents, especially when for use in any condition described herein) and in particular in a method of prophylaxis or treatment of the human or animal body, by therapy, or of diagnosis. The compounds and salts may be used wherever an ACF inhibitor and/or β-blocker is indicated. Thus, for example, the compounds of formula (I) and their physiologically acceptable salts may be used in the therapy of cardiovascular disorders, especially for the treatment of all forms of hypertension in both the long and the short term, (particularly that associated with high serum renin levels) and in general, for the treatment and prophylaxis of congestive heart failure. Compounds of formula (I) and their physiologically acceptable salts may also be used in the treatment of hyperaldosteronism, angina pectoris, glaucoma and migraine.

The amount of a compound of the invention or a physiologically acceptable salt thereof which is required to achieve the desired biological effect will of course depend on a number of factors, for example the specific compound chosen, the use for which it is intended, the mode of administration, and the recipient. In general, a daily dose is expected to lie in the range of from 1 ng to 100mg per day per kilogram bodyweight, eg 50ng-50mg, especially 500ng-5mg/kg/day. For example, an intravenous dose is for example in the range of from 10 ng to 1 mg/kg which may conveniently be administered as an infusion of from 0.1 ng to 50 μg per kilogram per minute. Infusion fluids suitable for this purpose for example contain from 0.01 ng to 100 μg, especially from 0.1 ng to 100 μg per millilitre. Unit doses for example contain from 100 ng to 100 mg of the active ingredient, for example ampoules for injection contain from 100 ng to 1 mg, and orally administrable unit dose formulations such as tablets or capsulesfor example contain from 0.001 to 50, especially 0.02 to 20 mg. In the foregoing passage, in the case of physiologically acceptable salts, weights indicated refer to the weight of the active ingredient ion, that is to say the ion derived from the compound of formula (I).

For use in the treatment or prophylaxis of the conditions referred to above, while the compounds of the invention and their physiologically acceptable salts may be used as the compound <u>per se</u>, they are preferably presented with an acceptable carrier therefor as a pharmaceutical formulation in accordance with the present invention. The carrier must of course be 'acceptable' in the sense of being compatible with the other ingredients of the formulation and not deleterious to the recipient thereof. The carrier may be a solid or a liquid, and

is preferably formulated with the compound as a unit-dose formulation, for example a tablet, which may contain from 0.05% to 95% by weight of compound. Other pharmacologically active substances may also be presented in formulations of the present invention as indicated above. The compounds of the invention and their physiologically accceptable salts may be incorporated in the formulations either in the form of an acid, salt or ester thereof, and the formulations may be prepared by any of the well-known techniques of pharmacy consisting essentially of admixture of the components of the formulation.

The formulations include those suitable for oral, rectal, topical, buccal (e.g. sub-lingual), parenteral (e.g. subcutaneous, intramuscular, intradermal or intranvenous) administration, although the most suitable route in any given case will depend on the nature and severity of the condition being treated, and on the nature of the particular compound of the invention or a physiologically acceptable salt thereof.

Formulations suitable for oral administration may be presented in discrete units such as capsules, cachets, lozenges or tablets each containing a predetermined amount of compound of the invention or a physiologically acceptable salt thereof; as a powder or granules; as a solution or a suspension in an aqueous liquid or a non-aqueous liquid; as an oil-in-water emulsion; or as a water-in-oil liquid emulsion. Such formulations may be prepared by any of the methods of pharmacy but all methods include the step of bringing into association the compound with the carrier which constitutes one or more accessory ingredients. In general they are prepared by uniformly and intimately admixing the compound with liquid or finely divided solid carriers or both, and then, if necessary, shaping the product into the desired presentation. For example, a tablet may be prepared by compression or moulding a powder or granules of the compound, optionally with one or more accessory ingredients. Compressed tablets may be prepared by compressing, in a suitable machine, the compound in a free-flowing form such as a powder or granules optionally mixed with a binder, lubricant, inert diluent, surface active or dispersing agent(s). Moulded tablets may be made by moulding in a suitable machine, the powdered compound moistened with an inert liquid diluent.

Formulations suitable for buccal (sub-lingual) administration include lozenges comprising a compound of the invention or a physiologically acceptable salt thereof in a flavoured basis, usually sucrose and acacia or tragacanth; and

pastilles comprising the compound in an inert basis such as gelatin and glycerin, or sucrose and acacia.

Formulations of the present invention suitable for parenteral administration conveniently comprise sterile aqueous preparations of a compound of the invention or a physiologically acceptable salt thereof, which preparations are preferably isotonic with the blood of the intended recipient. These preparations are preferably administered intravenously, although administration may also be effected by means of subcutaneous or intramuscular or intradermal injection. Such preparations may be conveniently prepared by admixing the compound with water and rendering the product sterile and isotonic with the blood. Injectable compositions according to the invention will generally contain 0.1 to 5% w/w of active ingredient.

Formulations suitable for rectal administration are preferably presented as unit-dose suppositories. These may be prepared by admixture of the compound of the invention or a physiologcially acceptable salt thereof with one or more of the conventional solid carriers, for example cocoa butter, and shaping of the resulting mixture. Formulations suitable for topical application to the skin preferably take the form of an ointment, cream, lotion, paste, gel, spray, aerosol or oil. Carriers which may be used include vaseline, lanoline,polyethylene glycols, alcohols and combinations thereof. The active ingredient is generally present in a concentration of from 0.1 to 15% w/w of the composition, for example from about 0.5 to about 2%.

The compounds of the invention and their physiologically acceptable salts may be prepared in any conventional manner and in accordance with the present invention, may for example be prepared by any method hereinafter described. In particular, the compounds of the invention and their physiologically acceptable salts may be prepared by any of the methods standard in the art of peptide chemistry and may comprise coupling the aryl moiety, the amino-(hydroxy)alkoxy side chain and the amino acids or derivatives thereof which together form the compounds of the invention, in any desired order. It will be appreciated that for example, the aryl moiety, side chain and the amino acids or their derivatives may be coupled in any order to form two separate chemical entities which, as a final process stage (which we may claim as an aspect of the present invention) are then coupled to form the compound of the invention or a precursor therefor and if appropriate, converting the precursor (which conversion we may also claim as an aspect of the present invention) to the

desired compound of the invention, and optionally if desired, converting the compound of the invention to another compound of the invention, and also optionally if desired, converting any compound of the invention so formed to a physiologically acceptable salt thereof.

Thus, according to the present invention, we also provide a process for the preparation of a compound of the invention or physiologically acceptable salt thereof by a method for the preparation of compounds of formula (I) and their physiologically acceptable salts as defined in the aforesaid European Patent Application No. 85306144.8, which method comprises reacting a compound $R^1$ with a compound $R^2$, wherein,

(a) $R^1$ represents a reactive derivative of a compound of formula (II)

R-(X) (II)

and $R^2$ represents a compound of formula (III)

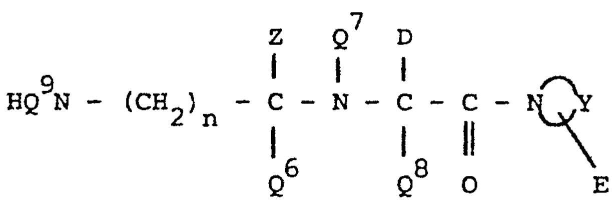

(III)

or a reactive derivative thereof; or

(b) $R^1$ represents a reactive derivative of a compound of formula (IV)

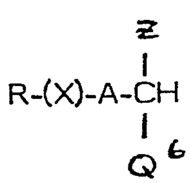

(IV)

and $R^2$ represents a reactive derivative of a compound of formula (V)

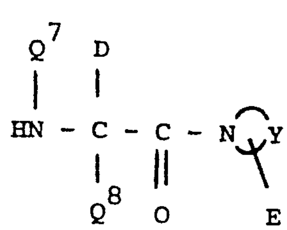

(V)

;or

(c) $R^1$ represents a compound of formula (VI)

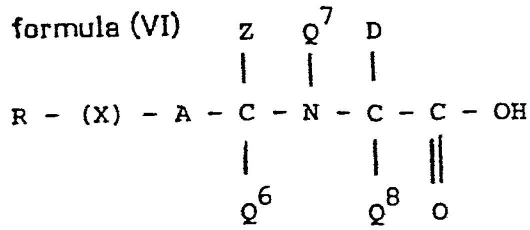

(VI)

or a reactive derivative thereof, and $R^2$ represents a compound of formula (VII)

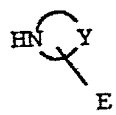

(VII)

or a reactive derivative thereof;

(wherein, in the compounds of formula (II) - (VII), R represents a group of formula (VIII)

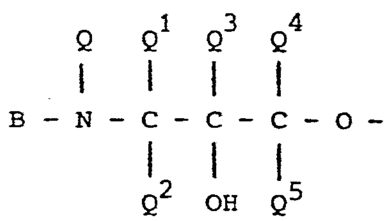

(VIII)

or R represents a reactive substituent on group (X), and in formulae (II) -(VIII), n, Q, $Q^1$-$Q^9$, A,B,D,E, 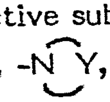, (X) and Z are as hereinbefore defined, and where appropriate, any of the compounds of formulae (II) - (VII) and the group of formula (VIII) include protected forms thereof);

and subsequently if desired, performing one or more of the following optional reactions in any desired order:-

(i) where the product of the reaction of compounds $R^1$ and $R^2$ is a precursor to a compound of formula (I) subjecting that precursor to such conditions and/or treating it with an appropriate agent or

agents, thereby to bring about consequent formation of a compound of formula (I);

(ii) converting any compound of formula (I) so formed to another compound of formula (I); and

(iii) converting any compound of formula (I) formed, to a physiologically acceptable salt thereof.

In formulae (II) - (VIII), reference to a reactive derivative of a compound of any such formula includes references to such compounds when substituted by one or more reactive moieties, and/or to such compounds when one or more moieties thereof are replaced by one or more reactive moieties.

In formula (III) above and throughout this specification, any of $-(CH_2)_n-$ groups forming part of a formula of an intermediate (eg as occuring hereinbelow in formulae (XII), (XX), (XXI), (XXIII) and (XXV)) are each independently optionally substituted by one or two $C_{1-4}$ alkyl groups.

The above optional reactions (i) - (iii) constitute further aspects of the present invention and may be claimed herein individually or in any desired combination.

Optional reaction (i) includes the situation wherein the precursor to the compound of formula (I) is an analogue of such a compound but wherein one or more moieties thereof are protected with a suitable protecting group, and the reaction comprises subjecting the precursor to such conditions and/or treating it with an agent or agents thereby to bring about deprotection with consequent formation of the desired compound of formula (I).

Details of the methods of peptide chemistry, and in particular suitable activating and protecting groups and of suitable reaction conditions for the above processes may be found in the following literature which is given purely by way of exemplification and which is intended to be neither exhaustive nor limiting:-

a) Schroder and Luebke, "The Peptides" (Academic Press) (1965).
b) Bellean and Malek, J.Am.Chem. Soc., 90, 165, (1968).
c) Tilak, Tetrahedron Letters, 849 (1970).
d) Beyerman, Helv. Chim. Acta., 56, 1729 (1973).

e) Stewart and Young, "Solid Phase Peptide Synthesis" (W.H.Freeman and Co.) (1969).

f) "Methoden der Organischen Chemie" (Houben-Weyl), 4th Edn., Vol.15, "Synthese von Peptiden", Parts 1 and 2 (Georg Thieme Verlag, 1974).

g) "The Peptides: Analysis, Synthesis, Biology", Gross, E. and Meienhofer, J. eds., Vols. 1 to 4 (Academic Press, 1979).

h) "Peptides: Syntheses, Physical Data", Voelter, W. and Schmid-Siegmann, E., Vols. 1 to 6 (George Thieme Verlag, 1983).

i) E. Atherton, M. J. Gait, R. C. Sheppard and B. J. Williams, Bioorganic Chem., 1979, 8, 351-370.

j) R. C. Sheppard, Chemistry in Britain, 1983, 402-414.

k) E. Atherton, E. Brown and R. C. Sheppard, J.C.S. Chem. Comm. 1981, 1151-1152.

l) T.W. Greene, Protective Groups in Organic Synthesis, Wiley, New York, 1981

m) G.C. Barrett (Ed.), The Chemistry and Biochemistry of the Amino Acids, 1st Edn., Chapman and Hall, London and New York, 1985.

As usual in peptide chemistry, different protecting groups may be used simultaneously on different moieties of a molecule to enable selective deprotection at different reaction stages, according to the reagents and reaction conditions in use. Thus for example, the protecting groups BOC (t-butoxycarbonyl) and TBDMS (t-butyldimethylsilyl) may be removed by treatment with a strong acid such as hydrochloric, preferably in an appropriate solvent such as methanol. The protecting group Z (benzyloxycarbonyl) may be removed by hydrogenation over palladium-carbon, preferably under acidic conditions, in a suitable solvent such as methanol. When it is necessary to protect a carboxy group such as defined for the groups E and Z above, this may conveniently be effected by formation of a corresponding simple ester such as the t-butyl ester and deprotection may be performed by acid hydrolysis.

Optional reaction (i) also includes the case wherein, in the precursor to the compound of formula (I), R represents a reactive substituent (or protected form thereof) on group (X), and the reaction comprises treating that product with an agent or agents serving the effect substitution of the group of formula (VIII) onto (X).

In that case, for the preparation of compounds of formula (I) wherein Q is hydrogen, the reactive substituent may be hydroxy and the precursor reacted with a compound of formula (IX)

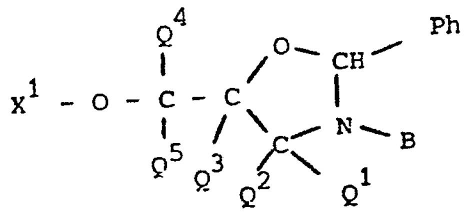

(IX)

(wherein $Q^1$-$Q^5$ and B are as hereinbefore defined, Ph represents phenyl and $X^1$ is an appropriate leaving group such as mesyl and the reaction is conveniently effected under basic conditions, followed by deprotection/de-cyclisation with an appropriate reagent such as trifluoroacetic acid.

Alternatively, the reactive substituent may be a group of formula (X)

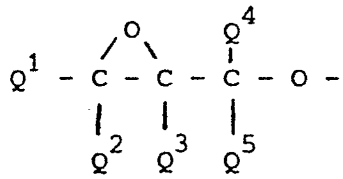

(X)

(wherein $Q^1$-$Q^5$ are as hereinbefore defined) and the reagent is an appropriate amine, the reaction conveniently being performed by heating in an appropriate solvent such as dioxan.

It will be appreciated that instead of substituting the group of formula (VIII) onto the ring or ring system (X) as part of optional reaction (i), as an aspect of the present invention, an equivalent subsitution may be made at any appropriate stage during the entire procedure for synthesis of the compounds of formula (I) and their physiologically acceptable salts. In particular, this substitution may be effected by following either of the procedures specified above in respect of the compounds of formulae (IX) and (X) but then in place of the precursor to the compound of formula (I) in which R represents a reactive substituent (or protected form thereof), one uses an appropriate intermediate including said group R, when R represents a reactive substituent (or protected form thereof). Examples of intermediates suitable for this purpose are those defined

hereinbelow by the formulae (XI), (XIII), (XV), (XVI), (XIX), (XXI), (XXVII) and (XXIX). Such procedures may, as appropriate, optionally require one or more steps of protection and/or deprotection as required.

Optional reaction (i) further includes the case, wherein a compound of formula (I) wherein Z represents an ester is formed by reaction of the corresponding compound of formula (I) wherein Z represents carbamoyl with the appropriate alcohol, in the presence of an acid catalyst, preferably of the ion exchange type, such as Amberlyst 15.

Optional reaction (ii) may comprise the case when in the compound of formula (I), F and/or Z represent(s) a carboxy group, converting the compound to a corresponding compound wherein F and/or Z represent(s) a carboxylic acid derivative, for example by treatment of the compound with an appropriate alcohol in the presence of a suitable acid such as sulphuric acid.

Optional reaction (ii) may also comprise the case wherein the compound of formula (I), F and/or (Z) represent(s) a carboxylic acid derivative, converting the compound to a corresponding compound wherein F and/or Z represent(s) a carboxy group, for example by hydrolysis, eg. under basic conditions, for example in the presence of sodium hydroxide.

Optional reaction (iii) may comprise reacting the compound of formula (I) thus formed, with an appropriate mineral or organic acid.

Specific sub-classes of the above process for preparing a compound of the invention or physiologically acceptable salt thereof according to the manner of preparation of the compounds of formula (I) and their physiologically acceptable salts, include the following:-

(A) as a preferred sub-class of reaction variant (a) above, for the preparation of compounds of formula (I) wherein k and m are both 1, reacting a compound of formula (XI)

$$R\text{-}(X)\text{-}R^3 \qquad (XI)$$

(wherein R and (X) are independently as hereinbefore defined or as appropriate, protected forms thereof, and $R^3$ represents carboxy or a

carboxylic acid derivative) with a compound of formula (XII)

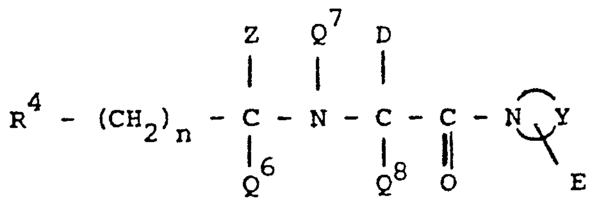

(XII)

(wherein n, D, F, Z, $Q^6$-$Q^8$ and N Y are independently as hereinbefore defined, or as appropriate, protected forms thereof, and $R^4$ represents a group of formula $-NHQ^9$ in which $Q^9$ is as hereinbefore defined);

(B) as a preferred sub-class of reaction variant (b) above, reacting a compound of formula (XIII)

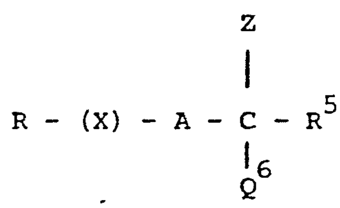

(XIII)

(wherein R, (X) A, $Q^6$ and Z are independently as hereinbefore defined, or as appropriate, protected forms thereof, and $R^5$ is as defined below in the definition of formula (XIV)) with a compound of formula (XIV)

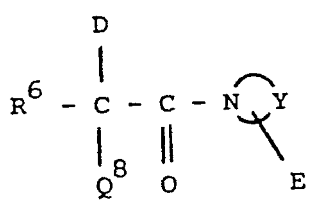

(XIV)

(wherein D,F, $Q^8$ and N Y are independently as hereinbefore defined, or as appropriate, protected forms thereof and one of $R^5$ (in formula (XIII) above) and $R^6$ is a group of formula $-NHQ^7$ and the other is a leaving group);

(C) as a preferred sub-class of reaction variant (c) above, reacting a compound of formula (VI) as hereinbefore defined, with a compound of formula (VII) as hereinbefore defined;

(D) as a preferred sub-class of optional reaction (i) above, reducing a compound of formula (XV)

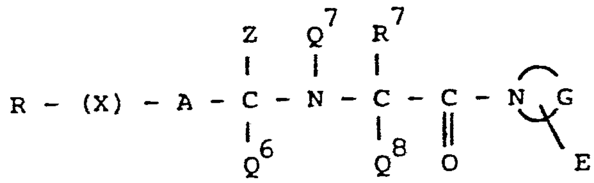

(XV)

(wherein R, (X), A, E and $Q^6$-$Q^8$ are independently as hereinbefore defined, or as appropiate, protected forms thereof, and $R^7$ and N G are independently selected from groups as defined above for D and N Y respectively and unsaturated forms thereof, provided at least one is in such an unsaturated form; and

(F) as another preferred sub-class of optional reaction (i) above, for the preparation of compounds of formula (I) wherein $Q^6$ and $Q^7$, or $Q^8$ are hydrogen, reducing a compound of formula (XVI)

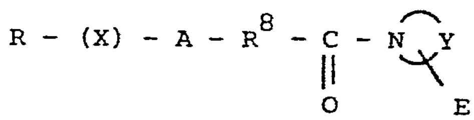

(XVI)

(wherein R, (X), A, F and N Y are independently as hereinbefore defined, or as appropriate, protected forms thereof, and $R^8$ is a group of formula (XVII)

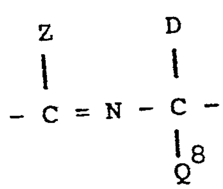

(XVII)

(wherein Z, D and $Q^8$ are as hereinbefore defined) or a group of formula (XVIII)

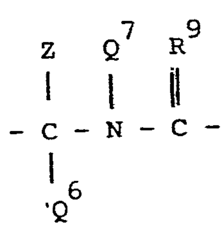

(XVIII)

(wherein Z, $Q^6$ and $Q^7$ and as hereinbefore defined and $R^9$ is an analogue of the group D as hereinbefore defined (other than hydrogen) and which has the same formula as D except that it lacks one hydrogen atom and so is linked to the $-NQ^7-C-$ moiety by a double bond));

(F) as a yet further preferred sub-class of optional reaction (i) above, for the preparation of compounds of formula (I) wherein Z represents carboxyl or carbamoyl, treating a compound of formula (XIX)

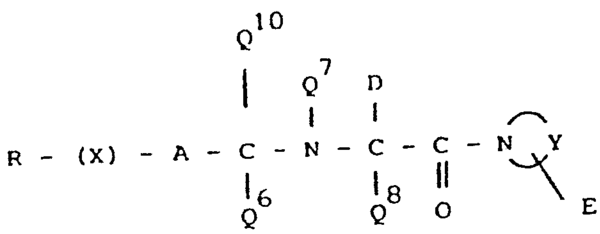

(XIX)

(wherein R, (X), A, D, F, $Q^6$-$Q^8$ and 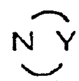 are independently as hereinbefore defined, or as appropriate, protected forms thereof and $Q^{10}$ represents an appropriate carboxyl or carbamoyl precursor) with an agent or agents serving to effect transformation of the precursor group $Q^{10}$ to a carboxyl or carbamoyl group;

and optionally if desired, effecting one or more of the aforesaid optional reactions (i)-(iii), or as appropriate, one or more further of said optional reactions, in any desired order.

Reaction (A) is conveniently performed using dicyclohexylcarbodiimide (DCCI) in the presence of 1-hydroxybenzotriazole (HOBT) to suppress racemisation, in a suitable solvent such as methylene chloride or DMF at a reduced temperature, eg in the range of from -15°C to 0°C, or up to room temperature as appropriate. Such a reaction protocol is hereinafter termed 'DCCI/HOBT' conditions.

Reaction (B) is conveniently performed at room temperature or with heating as appropriate, in a suitable solvent such as dichloromethane or DMF.

Reaction (C) is conveniently performed under DCCI/HOBT conditions, or by mixed anhydride coupling, which is a technique well known to those skilled in the art of peptide chemistry, but by way of example, may

comprise reacting the acid intermediate with isobutylchloroformate and N-methylmorpholine, followed by reaction with the amine intermediate, the reaction conveniently being performed at a reduced temperatue, eg. from -15°C to -25°C, to minimise racemisation.

Reaction (D) is conveniently performed by hydrogenation over a suitable catalyst, such as palladium on carbon, for example in a suitable solvent such as methanol.

In Reaction (D), one preferred class of compounds of formula (XV) are the compounds of formula (XVI) in which $R^8$ is a group of formula (XVIII) in which $R^9$ represents $CH_2$. Another preferred class of compounds of formula (XV) are those wherein 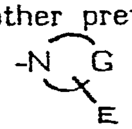 represents a ring having the formula 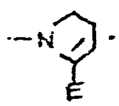.

Reaction (F) is also conveniently performed by hydrogenation for example by the method described in the preceeding paragraph. It will be appreciated that in this reaction, the intermediate of formula (XVI) may be formed *in situ* (for example by a method as described hereinbelow) and when in the compound of formula (XVI), the group $R^8$ has the formula (XVII) in which $Q^8$ represents hydrogen, the group of formula (XVII) may also have the resonance form (XVIIA)

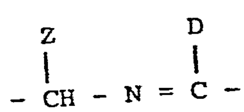

(XVIIA)

(wherein D and Z are independently as hereinbefore defined, or as appropriate, protected forms thereof), and when the group $R^8$ has the formula (XVIII) in which $Q^7$ represents hydrogen, the group (XVIII) may have the resonance form (XVIIIA)

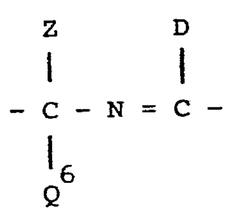

(XVIIIA)

(wherein D, Z and $Q^6$ are independently as hereinbefore defined, or as appropriate, protected forms thereof).

Reaction (F) may for example comprise hydrolysis of a compound of formula (XIX) in which $Q^{10}$ represents cyano. Such hydrolysis is conveniently performed by acid hydrolysis, eg in a suitable strong mineral acid such as hydrochloric, in a suitable solvent such as methanol.

Alternatively, Reaction (F) may comprise reaction of a compound of formula (XIX) wherein $Q^{10}$ is an appropriate carbonyl derivative with one or more suitable reagents serving to effect amination of the said carbonyl derivative. Thus for example, when $Q^{10}$ represents an activated carbonyl group such as an ester or an acid chloride, the compound of formula (XIX) may be reacted with ammonia, preferably under aqueous conditions. If the carbonyl derivative is carboxy, the compound of formula (XIX) may be treated with a reagent serving to effect formation of a corresponding activated acid, in the presence of, or followed by reaction with an appropriate reagent such as described above in respect of reactions with esters and acid chlorides.

The reagent serving to effect formation of a corresponding activated acid may be sulphur oxychloride, a phosphorus halide such as phosphorus tri- or pentachloride, a phosphorus oxyhalide such as the oxychloride, trifluoroacetic anhydride, an alkyl-(eg. ethyl-) chloroformate or any other suitable agent which will be apparent to those skilled in the art. A list of such reagents appears in Houben-Weyl (ref. (f) supra), Part 1, p.29. Conveniently, the reaction may be performed in a suitable solvent such as toluene, desirably in the presence of an appropriate catalyst such as dimethylformamide. Alternatively the reaction may be effected by reaction with an appropriate weak or volatile base such as ammonia, preferably by heating. Such a reaction conveniently may be effected using a stream of ammonia or by use of ammonia in situ in the presence of a dehydrating agent.

In reaction (B) and elsewhere in this specification, unless expressly indicated to the contrary, the or any leaving group may be selected from any of those known in the art of chemistry, for example as defined in J. March, Advanced Organic Chemistry, 3rd Edn., 1977, McGraw Hill, NY, p.187. Such leaving groups include halo (preferably chloro or bromo) mesyl (ie methanesulphonyloxy), tosyl (ie toluenesulphonyloxy) and triflate (ie trifluoromethanesulphonyloxy).

It will be appreciated that where any process according to the present invention does not inherently result in preparation of a desired individual isomer of a compound of formula (I) or a physiologically acceptable salt thereof, that process may involve separation of isomers, either of the end product itself or of any appropriate intermediate at any convenient stage of the reaction, for example, by means of column chromatography.

In process (A) supra, the compound of formula (XI) may be prepared according to the general method described in the aforesaid US Patent Specification no. 3,705,907. The compounds of formula (XII) may be prepared by methods generally analogous to those described in the above-mentioned European Patent Specificlation 0 012 401 and also in European Patent Specification no. EP 0 126 986 A1.

In process (B), compounds of formula (XIII) in which in the definition of A, k and m are both 1, may be prepared by reacting a compound of formula (XI) as hereinbefore defined with a compound of formula (XX)

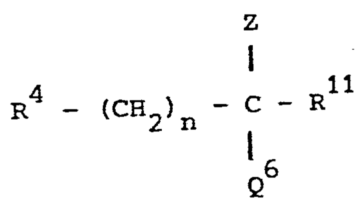

(XX)

(wherein $R^4$, n, Z and $Q^6$ are independently, as hereinbefore defined, or as appropriate, protected forms thereof, and when in the desired compound of formula (XIII), $R^5$ represents a group of formula $-NHQ^7$ as hereinbefore defined, then $R^{11}$ is a protected analogue of the group of formula $-NHQ^7$, and when in the desired compound of formula (XIII), $R^5$ represents a leaving group, then $R^{11}$ represents a protected hydroxy group, eg an alkoxy group such as t-butoxy) and in the case when $R^{11}$ represents a protected hydroxy group, treating the product of the reaction with an agent or agents serving to effect conversion of the protected hydroxy group to the desired leaving group. For example, when the desired leaving group is mesyl, the reaction product may be treated with a methanesulphonylhalide, such as the chloride.

Compounds of formula (XIII) in which in the definition of A, k and m are both 0, may be prepared by reacting a compound of formula (XXI)

$$R - (X) - (CH_2)_n - R^{12} \qquad (XXI)$$

(wherein R, (X) and n are independently as hereinbefore defined or as appropriate, protected forms thereof, and $R^{12}$ is an appropriate leaving group) with a compound of formula (XXII)

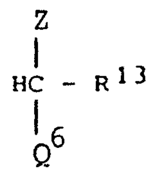

(XXII)

(wherein Z and $Q^6$ and $R^{11}$ are independently as hereinbefore defined, or as appropriate, protected forms thereof, and $R^{13}$ represents an appropriately protected amino group) and treating the reaction product with an agent or agents serving to effect deprotection of the protected amino group, and when in the desired compound of formula (XIII), $R^5$ represents a group of formula $NHQ^7$ in which $Q^7$ is a $C_{1-4}$ alkyl group, reacting the deprotected product with an appropriate alkylating agent or agents, and when in the desired compound of formula (XIII), $R^5$ represents a leaving group, reacting the deprotected product with an agent or agents serving to effect conversion of the amino group to said leaving group. For example, when the protected amino group $R^{13}$ is a group of formula PhCH=N- (in which Ph represents phenyl), this may be deprotected by treatment with a suitable base. The optional treatment of the deprotected product to convert the amino group to a leaving group may, for example, be effected according to the general method described in F.D. Thorset, Tet. Lett., 1982, 23, 1875-6.

The compounds of formula (XIII) in which in the definition of A, k and m are both 0, may also be prepared according to any of the methods described in Chapter 8 (pp 246 ff) of the G.C. Barrett (Ed.) reference (designated m) *supra*.

The compounds of formula (XIV) are simple dipeptides or trivial derivatives thereof and may be prepared by methods well known to those skilled in the art.

In process (C), compounds of formula (VI) wherein, in the definition of A, k and m are both 1, may be prepared by reacting a compound of formula (XI) as hereinbefore defined with a compound of formula (XXIII)

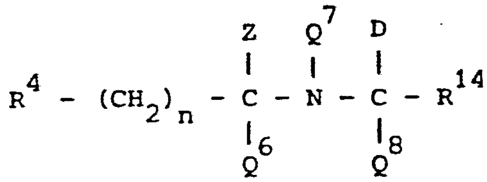

(XXIII)

(wherein $R^4$, n, D, Z and $Q^6$-$Q^8$ are independently as hereinbefore defined, or as appropriate, protected forms thereof and $R^{14}$ is a protected carboxy group) followed by deprotection of the group $R^{14}$.

Alternatively, compounds of formula (VI) wherein, in the definition of A, k and m are both 0, may be prepared by reaction of a compound of formula (XIII) as hereinbefore defined and wherein in the definition of A, k and m are both 0, with a compound of formula (XXIV)

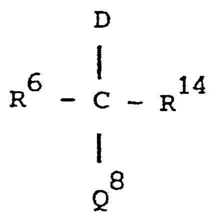

(XXIV)

(wherein $R^6$, D and $Q^8$ are independently as hereinbefore defined, or as appropriate, protected forms thereof, and $R^{14}$ is as hereinbefore defined) followed by deprotection of the group $R^{14}$.

The compounds of formula (VII) are simple amino acids or trivial derivatives thereof and may be prepared by methods well known to those skilled in the art.

In process (D), the compounds of formula (XV) wherein, in the definition of A, k and m are both 1, may be prepared by reaction of a compound of formula (XI) as hereinbefore defined, with a compound of formula (XXV)

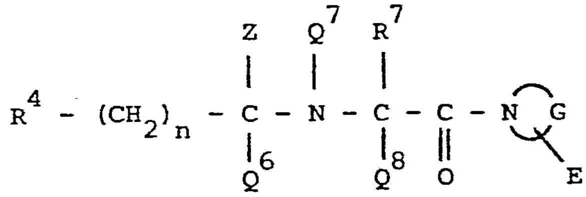

(XXV)

(wherein n, E, $R^4$, $R^7$, $Q^6$-$Q^8$ and 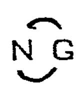 are independently as hereinbefore defined, or as appropriate, protected forms thereof), preferably under DCCI/HOBT conditions or by mixed anhydride coupling.

The compounds of formula (XV) wherein, in the definition of A, k and m are both 0, may be prepared by reaction of a compound of formula (XIII) as hereinbefore defined with a compound of formula (XXVI)

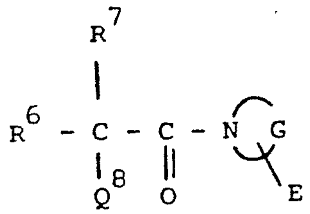

(XXVI)

(wherein E, $Q^8$, $R^6$, $R^7$ and N G are independently as hereinbefore defined, or as appropriate, protected forms thereof).

In process (F), the compounds of formula (XVI) may be prepared by reaction of a compound of formula (XXVII)

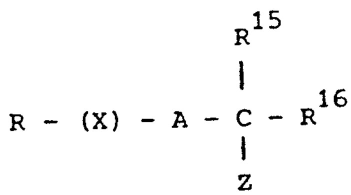

(XXVII)

(wherein R, (X), A and Z are independently as hereinbefore defined, or where appropriate, protected forms thereof, and either; (i) one of $R^{15}$ and $R^{16}$ is a group $Q^6$ as hereinbefore defined and the other is a group of formula $-NHQ^7$ as hereinbefore defined, or (ii) $R^{15}$ and $R^{16}$ together form =O) with a compound of formula (XXVIII)

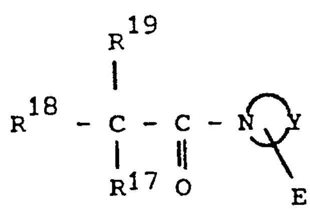

(XXVIII)

(wherein D, E and N Y are independently as hereinbefore defined, or as appropriate, protected forms thereof, and in the case (i) referred to in the definition of Formula (XXVII), $R^{17}$ and $R^{18}$ both represent =O and $R^{19}$ is a group D as hereinbefore defined, or in the case (ii), $R^{18}$ is a group of formula $-NHQ^7$ as hereinbefore defined and either $R^{17}$ is a group $Q^8$ as hereinbefore defined and $R^{19}$ is a group D as hereinbefore defined, or $R^{17}$ and $R^{19}$ together

represent a group $-R^9$ as hereinbefore defined. Preferably, this reaction is effected in a water-immiscible solvent such as toluene, benzene or chloroform, in the presence of an appropriate acid catalyst such as p-toluenesulphonic acid).

In process (F), the compounds of formula (XIX) wherein $Q^{10}$ represents cyano, may be prepared by reaction of a compound of formula (XXIX)

$$R - (X) - A - CHO \qquad (XXIX)$$

(wherein R, (X) and A are as hereinbefore defined, or as appropriate, protected forms thereof), with a compound of formula (V) as hereinbefore defined, in the presence of hydrogen cyanide, preferably formed in situ, for example by admixture of the reactants of formulae (XXIX) and (V) in the presence of an alkali metal cyanide (such as sodium or potassium cyanide) and an appropriate acid such as acetic acid, preferably in an appropriate solvent such as methanol. This reaction and the preparation of the intermediates of formulae (XXIX) and (V) may follow the general methodology described in J. Med. Chem., 1985, 28, 434-442.

The invention will now be described further by way of the following illustrative examples.

Example 1

N-(1(S)-Carboxy-5-[4-(2(S)-hydroxy-3-isopropylamino propoxy)-1H-indol-2-yl-carboxamido]pentyl)-(S)-alanyl-(S)-proline

(a) 2-Benzyloxybenzaldehyde

A mixture of salicylaldehyde (24.5g), benzyl bromide (34g), anhydrous potassium carbonate (15g) and acetone was stirred and refluxed for 5 hours. The solvent was removed in vacuo and the residue was partitioned between dilute alkali and ether. The ether layer was washed with water, dried ($MgSO_4$) and evaporated to give the product as

a viscous oil which slowly crystallised on standing. The solid thus obtained had m.p. 46°C.

(b) Ethyl azidoacetate

Ethyl bromoacetate (167g) and then tetrabutylammonium bromide (0.5g) were added to a solution of sodium azide (78g) in water (200ml). The mixture was vigorously stirred for 1 hour and then steam distilled. The oily product was separated and dried over anhydrous calcium chloride, yield 126g (97%).

(c) Methyl-4-benzyloxyindole-2-carboxylate

Sodium (9.2g) was dissolved in dry methanol (270ml) and the solution was cooled to 0°C. A mixture of 2-benzyloxybenzaldehyde from step (a) (42.4g) and ethyl azidoacetate from step (b) (51.6g) was then added. After stirring at 0-5°C for 5 hours, the mixture was poured onto ice and ammonium chloride (20g). The mixture was extracted with ether (600ml). The organic phase was washed with water, dried ($MgSO_4$) and evaporated in vacuo at 25°C. The yellow solid was dissolved in xylene (400ml) and the solution obtained was added dropwise during 2 hours to refluxing xylene (25ml). The solution was refluxed for a further hour, then left to stand overnight. 19g of methyl 4-benzyloxyindole-2-carboxylate, m.p. 193-194°C, was obtained. A specimen recrystallised from methanol had m.p. 195.5°C.

(d) Methyl 4-hydroxyindole-2-carboxylate

Methyl 4-benzyloxyindole-2-carboxylate from step (c) (4.2g) and 10% palladium on charcoal (0.5g) in methanol (100ml) were shaken with hydrogen for 1 hour. The catalyst was removed and the solvent evaporated. The residue was recrystallised from methanol/trichloroethylene (charcoal) to give 2.5g of methyl 4-hydroxyindole-2-carboxylate (87%) as colourless needles, m.p. 192-193°C.

(e) Methyl 4-(2(S)-oxiranylmethoxy)-1H-indole-2-carboxylate

A solution of methyl 4-hydroxyindole-2-carboxylate from step (d) (3.35g) in DMF (7ml) was added to a suspension of 60% sodium hydride-oil dispersion (704mg) in DMF (10ml) and the mixture stirred in an ice-bath until effervescence ceased. A solution of 2S-glycidyl tosylate (J. Org. Chem., 51, 3710 (1986)) (4.0g) in DMF (7ml) was added at $0^{o}C$; the mixture was heated to $50^{o}C$ for 3 hours, then cooled and the solvent removed *in vacuo*. The residue was subjected to chromatography on a 10 x 15cm column of silica using 2% $MeOH-CH_2Cl_2$ as eluant; *ca*. 100ml fractions were collected. Those fractions containing the product were combined and evaporated to leave a white solid (3.6g) which was recrystallised from isopropanol. Yield [illegible]5g, m.p. 141-144$^{o}$C, $[\alpha]_D = +8.75^{o}$ (1.0, $CHCl_3$).

(f) Methyl 4-[2(S)-hydroxy-3-isopropylamino]-1H-indole-2-carboxylate

The epoxide from step (e) (0.56g) was heated to $50^{o}C$ in a mixture of DMF (5ml), isopropylamine (5ml) and water (1ml) for 2 hours. The solvents were evaporated *in vacuo* and the residue stirred with EtOAc at $5^{o}C$ for 48 hours. The product was collected by filtration, yield 278mg. Further product was obtained from the mother liquor by chromatography on silica using $MeOH-CH_2Cl_2$-'880'$NH_3$ (90:10:1), yield 300mg. Chiral shift n.m.r. indicated that the product was >93% of a single enantiomer. M.p. 124-125$^{o}$C, $[\alpha]_D = -9.3^{o}$ (1.0, 1M HCl). Analysis: $C_{16}H_{22}N_2O_4$ requires C,62.73; H, 7.24; N, 9.14%, found: C, 62.48; H,7.41; N, 9.17%.

(g) Methyl 4-[3-(N-t-butoxycarbonyl-N-isopropylamino)-2(S)-hydroxypropoxy]-1H-indole-2-carboxylate.

The product from step (f) (0.64g) was dissolved in dry DMF (5ml), a solution of di-*t*-butyl pyrocarbonate (Fluka, 0.46g) in DMF (3ml) was added and the solution stirred at ambient temperature for 2.5 hours. Tlc indicated that the reaction was complete ($SiO_2$, $CHCl_3$-MeOH, 8:1). The solvent was evaporated and the residue subjected to chromatography

on silica using EtOAc-(40-60)petrol (1:2) as eluant. Those fractions containing the product were combined and evaporated and the residue crystallised under petrol. Yield 0.64g, m.p. 87-88°C, $[\alpha]_D$=+5.31° (1.0, MeOH). Analysis: $C_{21}H_{30}N_2O_6$ requires C, 62.05; H, 7.44; N, 6.89%; found: C, 62.08; H, 7.49; N, 6.77%.

(h) <u>4-[3-(N-t-butoxycarbonyl-N-isopropylamino)-2(S)-hydroxy-propoxy]-1H-indole-2-carboxylic acid</u>

The ester from step (g) (0.57g) was dissolved in MeOH (6ml) and 2M NaOH (2.1ml) added; the mixture was refluxed for 30min on a steam bath, cooled, diluted with water (5ml) and the MeOH evaporated <u>in vacuo</u>. The aqueous residue was filtered and the pH adjusted to 5 by dropwise addition of 10% citric acid. After stirring for 30min, the white precipitate was collected by filtration, washed with water and dried <u>in vacuo</u> over $P_2O_5$. Yield 0.42g, m.p. 170-172°C(dec.), $[\alpha]_D$=+4.9° (1.0, MeOH). Analysis: $C_{20}H_{28}N_2O_6$ requires C, 61.21; H, 7.19; N, 7.14%; found: C, 61.34; H, 6.97; N, 7.07%.

(i) <u>Methyl O-trifluoromethanesulphonyl-(R)-lactate</u>

This compound was prepared following the methodology described by Effenberger <u>et al</u>, Angew. Chem. Int. Ed., 1983, <u>95</u>, 50. Thus a solution of methyl R-lactate (Aldrich, 4.16g) and pyridine (2ml) in $CH_2Cl_2$ (10ml) was added dropwise to a stirred solution of trifluorom ethanesulphonic anhydride (Aldrich, 7.86ml) in $CH_2Cl_2$ (40ml) at <0°C. The reaction mixture was stirred and allowed to warm to <u>ca</u>. 10°C over 2 hours. The mixture was washed with water and brine, then dried over $MgSO_4$. The solvent was evaporated and the residue chromatographed on silica using $CH_2Cl_2$ as eluant. Fractions containing the product were evaporated to give a mobile colourless oil, 8.7g (containing solvent). This material was stored in a freezer and used directly in the subsequent step.

(j) Methyl N-[1(S)-t-butoxycarbonyl-5-N-benzyloxycarbonylamino-pentyl]-(S)-alaninate

The triflate from step (i) (3.6g) was dissolved in $CH_2Cl_2$ (15ml) and added dropwise to a solution of t-butyl $N^{\varepsilon}$-benzyloxycarbonyl-S-lysinate (J. Org. Chem., 28, 1251 (1963)) (8.05g) and triethylamine (5.6ml) in $CH_2Cl_2$ (40ml) at <-5°C over ca. 20min. The solution was stirred at 0°C for 1 hour, then allowed to warm to room temperature. The solvent was evaporated and the residue chromatographed on silica with EtOAc as eluant. Product fractions were concentrated to yield a gum, yield 5g. N.m.r. and mass spectra were consistent with the structure, $[\alpha]_D$=-24.96° (1.0, EtOAc).

(k) N-[1(S)-t-butoxycarbonyl-5- N-benzyloxycarbonylaminopentyl]-(S)-alanine

The ester from step (j) (5g) was dissolved in MeOH (15ml) and a solution of NaOH (0.66g) in water (7ml) added dropwise. The mixture was stirred at ambient temperaure for 5 hours, then diluted with water and the MeOH removed in vacuo. The residual aqueous solution was washed with ether (2x) and acidified by the dropwise addition of 1M HCl (16.5ml). The resulting white precipitate was collected by filtration, washed with water and dried over $P_2O_5$. Yield 3.78g. This material was converted to the HCl salt as follows: The solid was dissolved in warm dioxan (50ml), filtered and 3.4M HCl-dioxan (3.47ml) added; an oil was precipitated on addition of ether. The solvents were evaporated, the residue taken up in water and the residual dioxan removed by evaporation. The white precipitate was collected and dried. Yield 2g; a further 1.4g was obtained by freeze-drying the mother liquor. M.p. 140-141°C, $[\alpha]_D$ = -7.5° (1.0, dioxan).

(l) t-Butyl N-[1(S)-t-butoxycarbonyl-5-N-benzyloxycarbonyl-aminopentyl]-(S)-alanyl-(S)-prolinate

The acid from step (k) (3.4g) was dissolved in DMF (20ml) and N-methylmorpholine (0.46ml) was added. The solution was cooled in an ice-bath and 1-hydroxybenzotriazole (1.06g) was added followed by dicyclohexylcarbodiimide (1.79g). The mixture was stirred at $0^{o}C$ for 1 hour, then a solution of t-butyl-S-prolinate (J. Amer. Chem. Soc., 82, 3359 (1960)) (1.35g) in DMF was added. The reaction mixture was stirred at $4^{o}C$ for 18 hours, then filtered and concentrated in vacuo. The residue was taken up in EtOAc, filtered and the filtrate washed successively with 5% citric acid (3x), 5% $NaHCO_3$ (3x) and brine (1x) and dried over $MgSO_4$. The solvent was removed in vacuo and the residue chromatographed on silica with EtOAc-MeOH (99.5:0.5) as eluant. Product fractions were combined and evaporated to yield 2.2g, $[\alpha]_D = -57.8^{o}$ (1.03, $CHCl_3$).

(m) t-Butyl N-[1(S)-t-butoxycarbonyl-5-aminopentyl]-(S)-alanyl-(S)-prolinate

The compound from step (l) (5.19g) was dissolved in MeOH (50ml) under nitrogen and 10% Pd/C catalyst (0.5g) added. Hydrogen was bubbled through the stirred suspension for 2.5 hours when tlc indicated that the reaction was complete. Nitrogen was bubbled through the mixture and the catalyst removed by filtraton through celite. The filtrate was evaporated to leave the product as a colourless oil, 3.6g. $[\alpha]_D = -74.4^{o}$ (1.0, $CHCl_3$).

(n) t-Butyl N-[1(S)-t-butoxycarbonyl-5-(4-[3-(N-t-butoxycarbonyl-N-isopropylamino)-2(S)-hydroxypropoxy]-1H-indole-2-carboxamido)pentyl]-(S)-alanyl-(S)-prolinate

The indole carboxylic acid from step (h) (2g) was dissolved in DMF (20ml) and cooled in an ice-bath; 1-hydroxybenzotriazole 0.69g) was added followed by a solution of dicyclohexylcarbodiimide (1.05g) in

DMF (5ml). After stirring for 45 min at $0^{o}C$, a solution of the amine from step (i) (2.18g) in DMF (10ml) was added and the mixture stirred for 18 hours at ambient temperature. It was then filtered and concentrated *in vacuo*. The residue was taken up in EtOAc, filtered and the filtrate washed successively with 5% citric acid (3x), 5% $NaHCO_3$ (3x) and brine (1x) and dried over $MgSO_4$. The solvent was removed *in vacuo* and the residue (3.9g) chromatographed on silica with $CH_2Cl_2$-MeOH (92:8) as eluant. Product fractions were combined and evaporated to yield an off-white solid, 2.95g. $[\alpha]_D$=-46.7° (0.9, MeOH).
Analysis: $C_{42}H_{67}N_5O_{10}$ requires C, 62.90; H, 8.42; N, 8.74% Found: C, 63.16; H, 8.49; N, 8.63%.

(o) N-(1(S)-Carboxy-5-[4-(2(S)-hydroxy-3-isopropylaminopropoxy)-1H-indol-2-ylcarboxamido]pentyl)-(S)-alanyl-(S)-proline.

The compound from step (n) (2.9g) was dissolved in a mixture of trifluoroacetic acid-water-ethyl methyl sulphide (9:1:1, 2ml) and stirred at ambient temperature overnight. The solvents were evaporated and the residue taken up in water, washed with ether (2x), then freeze-dried. The residue was chromatographed on a column of DE52 cellulose (5x50cm), pre-equilibrated in 0.005M $NH_4HCO_3$, pH 8, and eluted with a linear gradient 31, 0.005-0.2M $NH_4HCO_3$, pH 8. Eluant fractions were monitored by HPLC (Zorbax C8, 0.2% TFA in 25% $H_2O$-MeCN). Those containing pure product were pooled and freeze-dried. The residue was desalted by adsorption to a Zorbax C18 column, washing with water and subsequent elution with MeOH. The eluate was evaporated and the residue dissolved in water, filtered and freeze-dried to leave the required product as a fluffy white solid. Yield 1.75g, $[\alpha]_D$=-30.2° (1.0, MeOH).
Analysis; $C_{29}H_{43}N_5O_8 \cdot 3.2H_2O$ requires C, 53.73; H, 7.70; N, 10.80%; found C, 53.87; H, 7.75; N, 10.66%.

Example 2 and 3

The following compounds in accordance with the invention were prepared in a manner analogous to the method of Example 1:

2. N-(1(S)-Carboxy-5-[5-(2,(R,S)-hydroxy-3-isopropylaminopropoxy)naphth-2-ylcarboxamido]pentyl)-(R,S)-alanyl-(S)-proline bistrifluoroacetate

3. N-(1(S)-Carboxy-5-[8-(2(R,S)-hydroxy-3-isopropylaminopropoxy)naphth-2--ylcarboxamido]pentyl)-(R,S)-alanyl-(S)-proline bistrifluoroacetate

The starting materials and reagents employed in the preparation of the compounds of Example 1 to 3 are available commercially or may be obtained by methods described in the chemical literature.

Pharmaceutical Formulations

In the following formulation examples, the "active compound" may be any compound of formula (I) or a physiologically acceptable salt thereof.

Example A:

TABLET

| | mg |
|---|---|
| Active Compound | 500 |
| Microcrystalline Cellulose | 100 |
| Polyvinylpyrrolidone | 10 |
| Sodium Starch Glycollate | 30 |
| Magnesium Stearate | 5 |
| | 645 |

Mix the active compound, the microcrystalline cellulose and the sodium starch glycollate. Granulate with the polyvinylpyrrolidone dissolved in alcohol. Dry mix in the magnesium stearate and compress (645 mg).

Example B:

CAPSULE

| | mg |
|---|---|
| Active Compound | 250 |
| Lactose | 150 |
| Starch | 25 |
| Magnesium Stearate | 5 |
| | 430 |

Mix the active compound, the lactose, the starch and the magnesium stearate. Fill into hard gelain capsules.

Example C:

SUPPOSITORY

| | mg |
|---|---|
| Active Compound | 500 |
| PEG 400 | 800 |
| PEG 6000 | 1700 |
| | 3000 |

Melt the PEG 6000. Mix in the PEG 400 followed by the active compound Pour into a suppository mould and allow to set.

Example D:

I/V INJECTION

| | | |
|---|---|---|
| Active Compound | | 500mg |
| Water for Injection B.P. | to | 5.0ml |

Dissolve the active compound in about nine tenths of the total volume of water for injection. When dissolution is complete, make to volume with more water for injection.

WMD/OLM/28th January 1986

Under aseptic conditions, sterilise the solution by filtration through a sterile, sterilising grade and pack the solution into previously sterilised ampoules, flushing the ampoules with nitrogen before and after filling.

Biological Examples

(a) β-Blockade in vitro

In an assay for β-adrenoceptor blockade in guinea pig left atria, a simple competitive blockade of isoprenaline gave the following results:

| | $pK_B$ |
|---|---|
| Compound of Example 1 | 7.0 |
| Compound of Example 2 | 5.4 |
| Compound of Example 3 | 5.3 |

(b) ACE inhibition in vitro

In an assay for angiotensin converting enzyme inhibition in guinea pig ileum, dose-dependent inhibition of contractile responses to Angiotensin I gave the following results:

| | $EC_{50}$ |
|---|---|
| Compound of Example 1 | 1.4nM |
| Compound of Example 2 | 13.5nM |
| Compound of Example 3 | 10.3nM |

A7.64 0234946

Claims

1. A compound of formula (I)

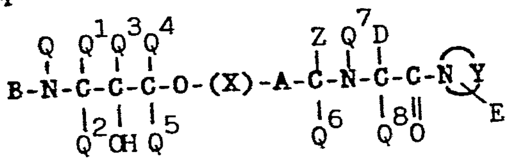

(I)

wherein:

Q and $Q^1$ - $Q^8$ are hydrogen;

A is a group of formula $-CONH(CH_2)_n-$, where n is 4 and $Q^9$ is hydrogen;

B is an isopropyl group;

E is a carboxyl group;

Z is a carboxyl or $C_{2-5}$ carboalkoxy group;

D is a methyl group;

(X) is a naphthyl or indolyl ring system; and

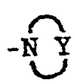 is a pyrrolidinyl ring;

and physiologically acceptable salts thereof;

with the condition that when (X) is a naphthyl ring system, the compound of formula (I) is:

N-(1(S)-carboxy-5-[5-(2(R,S)-hydroxy-3-isopropylaminopropoxy)naphth-2-ylcarboxamido]pentyl)-(R,S)-alanyl-(S)-proline, or

N-(1(S)-carboxy-5-[8-(2(R,S)-hydroxy-3-isopropylaminopropoxy)naphth-2-ylcarboxamido]pentyl)-(R,S)-alanyl-(S)-proline,

or a half ester (wherein Z is $C_{2-5}$ carboalkoxy) of either thereof,

and when (X) is an indolyl ring system, the compound of formula (I) is

N-(1(S)-carboxy-5-[4-(2(S)-hydroxy-3-isopropylaminopropoxy-1H-indol-2-yl-carboxamido]pentyl)-(S)-alanyl-(S)-proline,

or a half ester (wherein Z is $C_{2-5}$ carboalkoxy) thereof.

2. A compound of formula (I) as claimed in claim 1, or a physiologically acceptable salt thereof, for use in medical therapy.

3. A compound of formula (I) according to claim 1, or a physiologically acceptable salt thereof, for use in the prophylaxis or treatment of a cardiovascular disorder, hyperaldosteronism, glaucoma, or migraine.

4. A compound of formula (I) according to claim 1, or a physiologically acceptable salt thereof, for use in the prophylaxis or treatment of a cardiovascular disorder which is hypertension, congestive heart failure, myocardial infarction, or ischaemic heart disease.

5. A compound of formula (I) according to claim 1, or a physiologically acceptable salt thereof, for use in the prophylaxis or treatment of an ischaemic heart disease which is angina pectoris.

6. A compound of formula (I) according to claim 1, or a physiologically acceptable salt thereof, for use in the prophylaxis or treatment of a disease or disease symptom as defined in any of claims 6 to 8, which compound or salt is

N-(1(S)-carboxy-5-[5-(2(R,S)-hydroxy-3-isopropylaminopropoxy)naphth-2-ylcarboxamido]pentyl)-(R,S)-alanyl-(S)-proline,

N-(1(S)-carboxy-5-[8-(2(R,S)-hydroxy-3-isopropylaminopropoxy)naphth-2--ylcarboxamido]pentyl)-(R,S)-alanyl-(S)-proline, or

N-(1(S)-carboxy-5-[4-(2(S)-hydroxy-3-isopropylaminopropoxy)-1H-indol-2-ylcarboxamido]pentyl)-(S)-alanyl-(S)-proline,

or a half ester (as defined herein) of any thereof,

or a physiologically acceptable salt of any such compound.

7. Use of a compound of formula (I) as claimed in claim 1, or a physiologically acceptable salt thereof, in the prophylaxis or treatment of a disease or disease symptom as defined in any of claims 6 to 8.

8. Use according to claim 7, wherein the compound of formula (I) or a physiologically acceptable salt thereof is

N-(1(S)-carboxy-5-[5-(2(R,S)-hydroxy-3-isopropylaminopropoxy)naphth-2-ylcarboxamido]pentyl)-(R,S)-alanyl-(S)-proline,

N-(1(S)-carboxy-5-[8-(2(R,S)-hydroxy-3-isopropylaminopropoxy)naphth-2--ylcarboxamido]pentyl)-(R,S)-alanyl-(S)-proline, or

N-(1(S)-carboxy-5-[4-(2(S)-hydroxy-3-isopropylaminopropoxy)-1H-indol-2-ylcarboxamido]pentyl)-(S)-alanyl-(S)-proline,

or a half ester (as herein defined) of any thereof,

or a physiologically acceptable salt of any such compound.

9. A pharmaceutical formulation comprising a compound of formula (I) as claimed in claim 1, or a physiologically acceptable salt thereof, and an acceptable carrier therefor.

10. A process for the preparation of a compound of formula (I) as claimed in claim 1, or a physiologically acceptable salt thereof, which comprises coupling the aryl moiety (X), or any precursor thereto, the amino(hydroxy)alkoxy side chain B, or any precursor thereto, and the

aminoacids or aminoacid derivatives, or any precursor thereto, which together form the compound of formula (I), or a precursor thereto, and, when appropriate, converting the precursor compound to the compound of formula (I) and, if desired, converting any such compound of formula (I) to another compound of formula (I) and, if desired, converting any compound of formula (I) so formed to a physiologically acceptable salt thereof.

WMD/OLM/29th January 1986

Claims

1. A process for the preparation of a compound of formula (I)

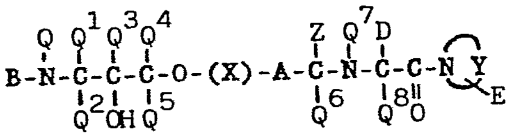 (I)

wherein:

Q and $Q^1$ - $Q^8$ are hydrogen;

A is a group of formula $-CONH(CH_2)_n-$, where n is 4 and $Q^9$ is hydrogen;

B is an isopropyl group;

E is a carboxyl group;

Z is a carboxyl or $C_{2-5}$ carboalkoxy group;

D is a methyl group;

(X) is a naphthyl or indolyl ring system; and

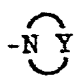 is a pyrrolidinyl ring;

and physiologically acceptable salts thereof;

with the condition that when (X) is a naphthyl ring system, the compound of formula (I) is:

N-(1(S)-carboxy-5-[5-(2(R,S)-hydroxy-3-isopropylaminopropoxy)naphth-2-yl carboxamido]pentyl)-(R,S)-alanyl-(S)-proline, or

N-(1(S)-carboxy-5-[8-(2(R,S)-hydroxy-3-isopropylaminopropoxy)naphth-2-ylcarboxamido]pentyl)-(R,S)-alanyl-(S)-proline,

or a half ester (wherein Z is $C_{2-5}$ carboalkoxy) of either thereof,

and when (X) is an indolyl ring system, the compound of formula (I) is

N-(1(S)-carboxy-5-[4-(2(S)-hydroxy-3-isopropylaminopropoxy)-1*H*-indol-2-yl-carboxamido]pentyl)-(S)-alanyl-(S)-proline,

or a half ester (wherein Z is $C_{2-5}$ carboalkoxy) thereof;

the process comprising coupling the aryl moiety (X), or any precursor thereto, the amino(hydroxy)alkoxy side chain, or any precursor thereto, and the aminoacids or aminoacid derivatives, or any precursor thereto, which together form the compound of formula (I), or a precursor thereto, and, when appropriate, converting the precursor compound to the compound of formula (I) and, if desired, converting any such compound of formula (I) to another compound of formula (I) and, if desired, converting any compound of formula (I) so formed to a physiologically acceptable salt thereof.

2. A process according to claim 1, which comprises reacting a compound $R^1$ with a compound $R^2$, wherein:

(a) $R^1$ is compound of formula (II)

R-(X) (II)

and $R^2$ is a compound of formula (III)

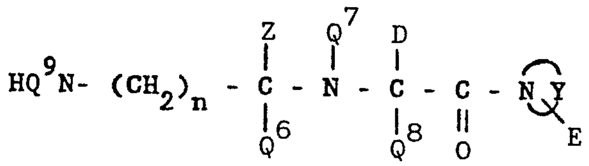

(III)

;or

(b) $R^1$ is a compound of formula (IV)

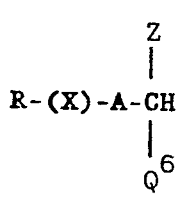

(IV)

A764 0234946

and $R^2$ is a compound of formula (V)

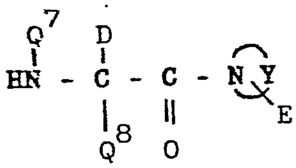

(V)

;or

(c) $R^1$ is a compound of formula (VI)

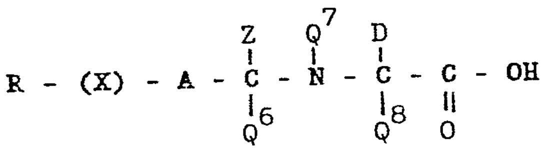

(VI)

and $R^2$ is a compound of formula (VII)

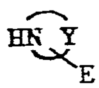

(VII)

wherein, in the compounds of formulae (II) to (VII), R is a group of formula (VIII)

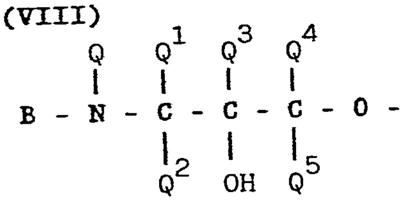

(VIII)

or R is a reactive substituent on group (X), and in formulae (II) to (VIII), n, Q, $Q^1$ - $Q^9$, A,B,D,E, 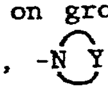, (X) and Z are as hereinbefore defined, and where appropriate, any of the compounds of formulae (II) to (VII) and the group of formula (VIII) include protected forms thereof;

and subsequently, if desired, performing one or more of the following optional reactions in any desired order:

(i) where the product of the reaction of compounds $R^1$ and $R^2$ is a precursor to a compound of formula (I), subjecting that precursor to such conditions and/or treating it with an appropriate agent or agents to bring about consequent formation of a compound of formula (I);

(ii) converting any compound of formula (I) so formed to another compound of formula (I); and

(iii) converting any compound of formula (I) to a physiologically acceptable salt thereof.

3. A process according to claim 1 or 2, which comprises:

(A) reacting a compound of formula (XI)

$R-(X)-R^3$ (XI)

wherein R and (X) are as hereinbefore defined, or, as appropriate, protected forms thereof, and $R^3$ represents carboxyl or a carboxyl derivative, with a compound of formula (XII)

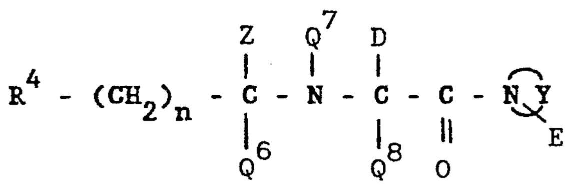 (XII)

wherein n, D,E,Z, $Q^6$-$Q^8$ and -N Y are as hereinbefore defined, or, as appropriate, protected forms thereof, and $R^4$ represents a group of formula $-NHQ^9$ in which $Q^9$ is as hereinbefore defined; or

(B) reacting a compound of formula (XIII)

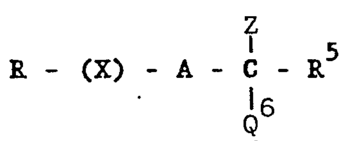 (XIII)

wherein R, (X) A,$Q^6$ and Z are as hereinbefore defined, or, as appropriate, protected forms therof, and $R^5$ is as defined below in the definition of formula (XIV), with a compound of formula (XIV)

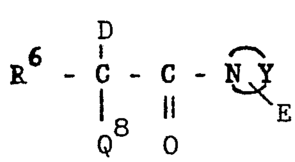 (XIV)

wherein D,E, $Q^8$ and N Y are as hereinbefore defined, or, as appropriate, protected forms thereof, and one of $R^5$ (in formula (XIII) above) and $R^6$ is a group of formula $-NHQ^7$ and the other is a leaving group; or

(C) reacting a compound of formula (VI) as hereinbefore defined, with a compound of formula (VII) as hereinbefore defined; or

(D) reducing a compound of formula (XV)

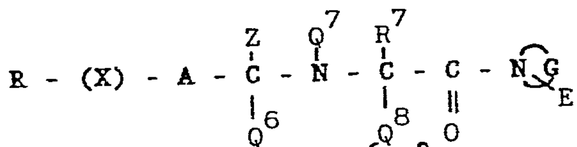

(XV)

wherein R, (X), A,E and $Q^6$-$Q^8$ are as hereinbefore defined, or, as appropriate, protected forms thereof, and $R^7$ and N G are independently selected from groups as defined above for D and N Y respectively and unsaturated forms thereof, provided at least one is such an unsaturated form; or

(E) reducing a compound of formula (XVI)

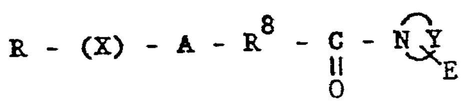

(XVI)

wherein R, (X), A, E and -N Y are as hereinbefore defined, or, as appropriate, protected forms thereof, and $R^8$ is a group of formula (XVII)

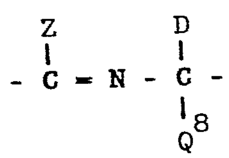

(XVII)

wherein Z, D and $Q^8$ are as hereinbefore defined,

or a group of formula (XVIII)

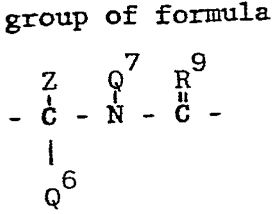

(XVIII)

wherein Z, $Q^6$ and $Q^7$ and as hereinbefore defined and $R^9$ is an analogue of the group D as hereinbefore defined (other than hydrogen) which has the same formula as D except that it lacks one hydrogen atom and is linked to the $-NQ^7$-C- moiety by a double bond; or

(F) treating a compound of formula (XIX)

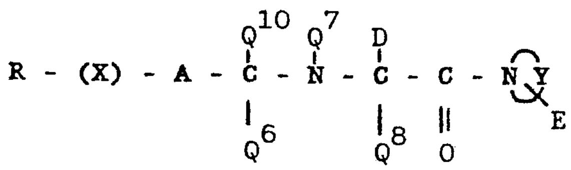

(XIX)

wherein R, (X), A,D,E, $Q^6$-$Q^8$ and -N Y are as hereinbefore defined, or, as appropriate, protected forms thereof, and $Q^{10}$ represents an appropriate carboxyl precursor, with an agent or agents serving to effect transformation of the carboxyl precursor to a carboxyl group;

and, if desired, effecting one or more of the optional reactions (i)-(iii) defined in claim 2, or, as appropriate, one or more further of said optional reactions, in any desired order.

4. A process according to claim 3(A), wherein the reaction is performed using dicyclohexylcarbodiimide (DCCI) in the presence of 1-hydroxybenzotriazole (HOBT) to suppress recemisation, in a suitable solvent and at a temperature of from -15°C to room temperature.

5. A process according to claim 3(B), wherein the reaction is performed at room temperature or with heating as appropriate, in a suitable solvent.

6. A process according to claim 3(C), wherein the reaction is performed under the DCCI/HOBT conditions defined in claim 4, or by mixed anhydride coupling.

7. A process according to claim 3(D) to (E), wherein the reaction is performed by hydrogenation over a suitable catalyst.

WMD/OLM/29th January 1987

8. A process according to claim 3(F), wherein the reaction comprises hydrolysis of a compound of formula (XIX) in which $Q^{10}$ is cyano, or reaction of a compound of formula (XIX) in which $Q^{10}$ is an appropriate carbonyl derivative with one or more suitable agents serving to effect transformation of the carbonyl derivative to a carboxyl group.

9. A process according to any of claims 1 to 8, wherein the compound of formula (I) or a physiologically acceptable salt thereof so prepared, is;

N-(1(S)-carboxy-5-[5-(2(R,S)-hydroxy-3-isopropylaminopropoxy)naphth-2--ylcarboxamido]pentyl)-(R,S)-alanyl-(S)-proline,

N-(1(S)-carboxy-5-[8-(2(R,S)-hydroxy-3-isopropylaminopropoxy)naphth-2--tlcarboxamido]pentyl)-(R,S)-alanyl-(S)-proline, or

N-(1(S)-carboxy-5-[4-(2(S)-hydroxy-3-isopropylaminopropoxy)-1H-indol-2-ylcarboxamido]pentyl)-(S)-alanyl-(S)-proline,

or a half ester (as defined herein) of any thereof,

or a physiologically acceptable salt of any such compound.

10. A process for the preparation of a pharmaceutical formulation by:

a) preparing a compound of formula (I), or a physiologically acceptable salt thereof, by a process according to any of claims 1 to 9; and

b) admixing the resulting product with an acceptable carrier therefor.

WMD/OLM/29th January 1987